# EUROPEAN PATENT APPLICATION

(11) **EP 1 442 658 A1**
(43) Date of publication of application: **04.08.2004**
(21) Application number: 02768048.7
(22) Date of filing: 25.09.2002
(51) Int. Cl.: A01K 67/04, A23K 1/16, A23K 1/18

(54) **FEED FOR ELEVATING VIRAL INFECTION RATIO IN SILKWORM AND METHOD OF VIRAL INOCULATION USING THE FEED**

(30) Priority: 04.10.2001 JP 2001308603
(71) Applicant: NATIONAL INSTITUTE OF AGROBIOLOGICAL SCIENCES, Tsukuba-shi, Ibaraki 305-8602 (JP)
(72) Inventor: ARAKAWA, Toru, Tsukuba-shi, Ibaraki 305-0051 (JP); FURUTA, Yoji, Tsukuba-shi, Ibaraki 305-0025 (JP); KUBOMURA, Yasue, Ryugasaki-shi, Ibaraki 301-0855 (JP); KATO, Masao, Tsukuba-shi, Ibaraki 305-0861 (JP); HAYASAKA, Shoji, Ushiku-shi, Ibaraki 300-1207 (JP); MIYAZAWA, Mitsuhiro, Tsukuba-shi, Ibaraki 305-0035 (JP)
(74) Representative: Hinkelmann, Klaus, Dr.
(86) International application number: PCT/JP2002/009854
(87) International publication number: WO 2003/030637

(57) **Abstract**

There is provided a process for producing a feed for elevating a rate of infection with a recombinant virus in a large number of silkworms, and a method of simultaneously inoculating orally a recombinant virus into a large number of silkworms. A process for producing a feed for elevating a rate of infection with a recombinant virus in a large number of silkworms includes mixing a powdery synthetic feed with a distilled water in which Tinopal UNPA-GX is dissolved at a final concentration of 0.2 to 0.4% by weight, grinding the mixture by stirring, putting it into a heat-resistant polyethylene bag, and heating it in an autoclave; and a method of simultaneously inoculating orally a recombinant virus to a large number of silkworms reared on a silkworm basket is characterized by including supplying a feed produced by the above process to a large number of 5th-instar silkworms reared on a silkworm basket, and, at 20 to 24 hours later, placing a net carrying a feed coated with a recombinant virus-containing solution thereon on the silkworm basket, thereby supplying the feed coated with the recombinant virus.

## Description

### Technical Field

The present invention relates to a process for producing a feed for elevating a rate of infection with a virus in silkworms and a method of efficiently inoculating a virus into silkworms using the feed.

### Background Art

Recently, a technique of producing a useful protein in silkworms using genetic recombination technology has been used in practice. For example, methods using a silkworm nuclear polyhedrosis virus as a vector are known (JP Patent Publication (Kokai) Nos. 61-9288, and 62-208276, and so on). These methods include preparing a recombinant virus by replacing a polyhedrin gene contained in a silkworm nuclear polyhedrosis virus with a gene encoding another useful substance such as interferon; inoculating the recombinant virus into 5th instar silkworms to infect the silkworms with the recombinant virus; and, 4 to 8 days later, collecting, separating and purifying the useful substance which has been produced in silkworm cells and secreted into the body fluid during the growth process of the recombinant virus.

Since a silkworm used as a host is genetically closer to a human than Escherichia coli and yeasts conventionally used as hosts, such a genetic recombination technique using silkworms is preferable in view of the activity and antigenicity of the produced useful substance.

### Disclosure of the Invention

However, a conventional genetic recombination process for producing a useful substance by use of silkworms has a big problem. When a recombinant virus is inoculated into silkworms in accordance with a conventional process, use is made of a method of injecting silkworms with a virus one by one or a method of orally inoculating a virus into silkworms just entering the 5th instar after they are treated at a low temperature of 5°C for 24 hours. However, in the former method, a recombinant virus cannot be inoculated into a large number of silkworms at a time. In addition, well-trained skill and time are required since inoculation must be performed so as not to damage various organs of silkworms. Further in the latter method, time and effort are required for loading silkworms into a low-temperature room and unloading therefrom. Therefore, the conventional methods are not always suitable for inoculating a recombinant virus into a large number of silkworms.

The present invention provides a process for producing a feed capable of elevating a rate of infection with a virus in silkworms, the method which makes it possible to inoculate the virus efficiently into a large number of silkworms without labor and drastically reduce the number of workers. The present invention also provides a method of infecting silkworms with a recombinant virus by using the feed.

The present inventors have made intensive studies. As a result, they found that a large number of silkworms can be infected with a virus at a time with minimum time and labor by providing the silkworms with a feed that is prepared by mixing a synthetic feed for silkworms in water containing a fluorescent whitener, grinding the mixture by stirring, heating it in an autoclave; feeding the silkworms with the autoclaved feed; and feeding the silkworms with a feed containing the virus.

More specifically, the present invention is summarized as follows.
(1) A process for producing a feed for elevating a rate of infection with a recombinant virus in silkworms, comprising: mixing a powdery synthetic feed with distilled water in which Tinopal UNPA-GX is dissolved at a final concentration of 0.2 to 0.4% by weight of the feed; grinding by stirring; putting the mixture into a heat-resistant polyethylene bag; and heating the mixture in an autoclave.
(2) The process for producing a feed for elevating a rate of infection with a recombinant virus in silkworms according to item (1), comprising: mixing a powdery synthetic feed with distilled water in which Tinopal UNPA-GX is dissolved at a final concentration of 0.3% by weight of the feed; grinding by stirring; putting the mixture into a heat-resistant bag; and heating the mixture in an autoclave.
(3) The process for producing a feed for elevating a rate of infection with a recombinant virus in silkworms according to item (1) or (2), in which the heating in an autoclave is performed at 100 to 121°C for 10 to 30 minutes.
(4) A method of simultaneously inoculating orally a recombinant virus into a large number of silkworms reared on a silkworm basket, comprising: feeding a large number of 5th instar silkworms reared on a silkworm basket with the feed produced by the process according to item (1); and mounting, at 20 to 24 hours later, a net carrying a feed coated with a recombinant virus-containing solution thereon, on the silkworm basket, thereby giving the feed coated with a recombinant-virus containing solution.
(5) The method of simultaneously inoculating orally a recombinant virus into a large number of silkworms according to item (4), comprising transferring silkworms fed with a recombinant virus and placed on the net onto a normal feed, together with the net.

The present invention will be described in detail.

### 1. Silkworm, virus and synthetic feed for silkworm used in the invention

The silkworm to be used in the present invention is not particularly restricted and a widely-used silkworm strain may be used. Examples of such a silkworm strain include crossbreeds such as Japanese No. 137 × Chinese No. 146, Shuko × Ryuhaku, Kinshu × Syowa, and Fuyo × Tokai. Furthermore, silkworms of the 5th instar, particularly, silkworms on day 1 of the 5th instar are preferable.

The feed for improving a rate of infection with a virus in silkworms of the present invention may be prepared using a commercially available synthetic feed such as Silkmate (trademark: Nosan Corporation) as a base. As the synthetic feed, a powdery feed is desirable; however, a pellet feed may be used after grinding. A powdery synthetic feed is mixed with distilled water containing Tinopal UNPA-GX at a final concentration of 0.2 to 0.4% by weight of the feed and grinding by stirring sufficiently. The mixture is then put into a heat-resistant polyethylene bag and subjected to treatment by an autoclave. In this manner, it is possible to obtain a synthetic feed of the present invention containing Tinopal for elevating a rate of infection with a virus in silkworms. The material for the heat-resistance polyethylene bag used herein is not restricted; for example, polyethylene terephthalate (PET), high density polyethylene (HDPE, MDPE), cast polypropylene (CPP) may be used.

The amount of virus to be administered to silkworms varies depending upon the type of recombinant virus to be used and the useful protein to be produced. For example, when a silkworm nuclear polyhedrosis virus (BmNPV) is used, a feed to which a 0.1% solution of the supernatant centrifugally obtained from the body fluid of silkworms infected with the polyhedrosis virus is applied and fed to silkworms for several hours.

Examples of the recombinant virus to be used in the present invention include a recombinant virus obtained by integrating a gene encoding a useful protein into a constitutional gene for a polyhedral protein of silkworm nuclear polyhedrosis virus DNA (BmNPV DNA). A recombinant virus may be prepared in accordance with a conventional method. More specifically, a polyhedral protein expression promoter (plasmid) of a silkworm nuclear polyhedrosis virus is prepared. A gene encoding a desired useful protein is integrated downstream of the promoter to prepare a transfer vector. Subsequently, established silkworm cells are co-transfected with the transfer vector and a silkworm nuclear polyhedrosis virus previously cloned. The transfected recombinant virus is cultured to proliferate in the same established silkworm cells. The recombinant virus may be obtained by destroying the established silkworm cells.

### 2. Inoculation of virus into silkworms and rearing method

According to the method of inoculating a virus into silkworms and the rearing method of the silkworm of the present invention, a large number of silkworms can be efficiently reared and inoculated with a virus at a time. The number of silkworms can be increased or decreased as required. Several thousands to several tens thousands of silkworms can be reared at a time.

A large number of silkworms are placed on a silkworm rearing bed (silkworm basket) and the like, and reared while appropriately controlling the temperature and humidity and feeding the synthetic feed containing Tinopal mentioned above to silkworms on the rearing bed. The synthetic feed is, desirably given herein in the amount that can be consumed by silkworms completely in 20 to 24 hours, for example, 1,400 g per 1,000 5th-instar silkworms. Subsequently, the feed coated with a virus is given to silkworms previously fed with a Tinopal-containing synthetic feed. The feed herein is desirably given in the amount that can be consumed by silkworms in several hours, for example, in 6 hours. More specifically, the amount is about 600 g per 1,000 5th-instar silkworms. The feed is preferably placed on a net which silkworms pass through and the net is mounted in its entirety on the rearing bed where silkworms are reared. By virtue of this method, the time and effort for transferring silkworms can be eliminated. In addition, since silkworms that have consumed the virus-coated feed are placed on the net, a large number of silkworms can be transferred at a time entirely by the net. Thereafter, silkworms fed with the virus-coated feed are transferred entirely by the net onto a rearing bed which carries feeds for several days thereon, and allowed to grow. Predetermined days later, a desired protein can be stably obtained. For example, when silkworms are reared at a constant temperature of about 25°C using a silkworm nuclear polyhedrosis virus as a recombinant virus, the silkworm larvae infected with polyhedrosis show an intrinsic symptom of nuclear polyhedrosis (grasserie) on day 5 after administration. In this way, the production of a useful substance proceeds.

A desired protein produced in a silkworm body is recovered and separated by taking the body fluid from silkworms on days 4 to 6 after infection when a desired protein is accumulated in the largest amount in a silkworm body, and separating the body fluid by a separation means such as centrifugal separation or column chromatography, thereby obtaining and purifying a useful protein. As another means, there is a method including mincing silkworms, extracting the minced silkworms with an aqueous SDS solution, aqueous urea solution, alkaline aqueous solution, or acidic aqueous solution, and separating and recovering a useful protein by a conventional process. As a still another means, there is a method including suspending the silkworms minced in a phosphate buffer, ultrasonically treating the suspended solution, and adding a substance, such as blood cells, specifically binding to a useful protein, to the solution mixture, thereby obtaining the useful protein.

### Brief Description of the Drawings

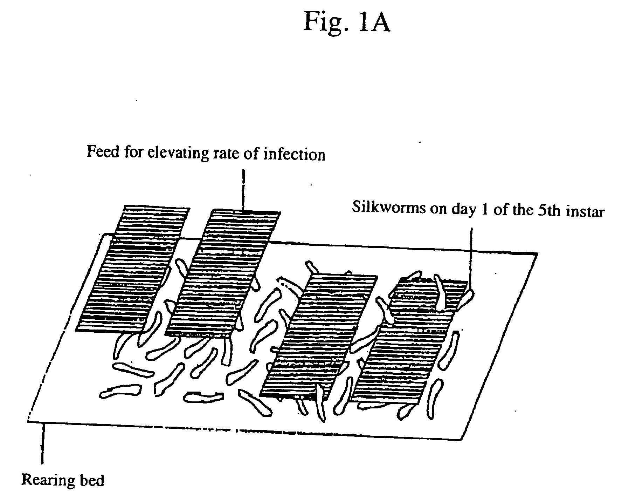

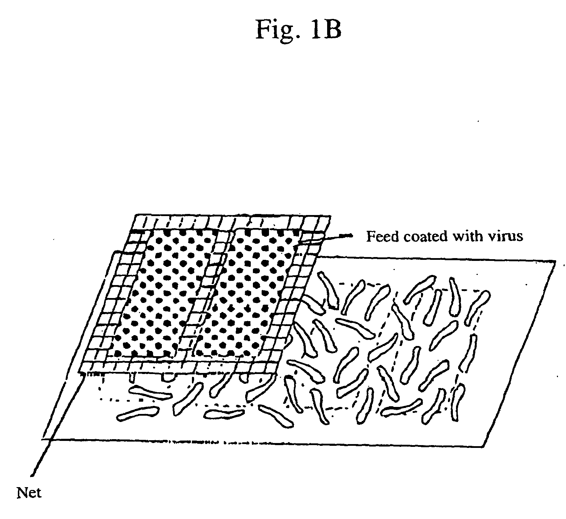

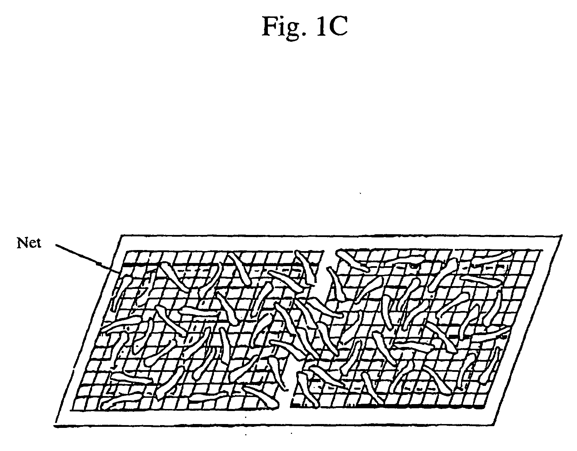

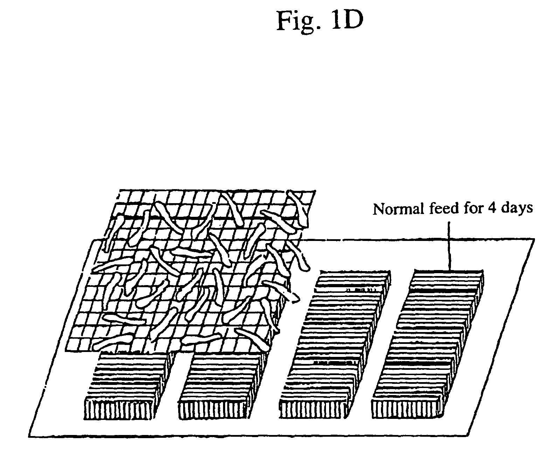

Figure 1 is an illustration showing a method of orally inoculating a virus into a large number of silkworms by using a feed containing Tinopal and rearing them. Figure 1A is an illustration showing a picture of placing silkworms on day 1 of the 5th instar and a feed for elevating a rate of infection on a rearing bed. Figure 1B is an illustration showing a picture where a net carrying a feed coated with a virus thereon is mounted on silkworms which have consumed a feed for elevating a rate of infection. Figure 1C is an illustration of showing a picture of silkworms which have consumed a feed coated with a virus. Figure 1D is an illustration of showing a picture where silkworms which have consumed a virus and placed on a net are transferred onto feeds for 4 days on a rearing bed.

### Best Mode for Carrying Out the Invention

The present invention will be described in detail below by way of Examples, which should not be construed as limiting the scope of the present invention.

### [Example 1] Production of feed for elevating a rate of infection of silkworm with a virus

One thousand grams of a powdery synthetic feed (commercially available Silkmate® manufactured by Nosan Corporation) was mixed with 2,700 ml of distilled water containing a fluorescent whitener (commercially available Tinopal UNPA-GX® manufactured by Sigma) at a final concentration of 0.3% by weight of the feed. The resultant mixture was grinded by stirring, put into a heat-resistant polyethylene bag, and heated in an autoclave for 20 minutes. The heated synthetic feed containing Tinopal was stored at 4°C.

### [Example 2] Inoculation of virus into silkworms

One thousand silkworms on day 1 of the 5th instar were placed on a rearing bed and a Tinopal-containing feed for one day prepared in Example 1 was placed on the bed and fed to the silkworms (Figure 1a). After silkworms consumed the feed for 20 to 24 hours, a normal feed whose entire surface has been coated with a BmNPV solution (the body fluid of silkworms with nuclear polyhedrosis virus diluted with distilled water to a concentration of 0.1%) by use of a wash bottle, was placed on a net having a large mesh size sufficient to pass silkworms. The net was mounted on the silkworms on the rearing bed entirely by the net (Figure 1b). At 6 hours later when the silkworms completely consumed the BmNPV-coated feed, the silkworms were moved from the rearing bed to the net (Figure 1c). Then, the net carrying silkworms thereon was mounted on normal feed for 4 days, which was placed on another rearing bed (Figure 1d). A cover was placed on the rearing bed to prevent the feed from drying and silkworm from creeping out, and stood it alone for 4 days. The amount of feeds and time required for infecting 1,000 silkworms of the 5th instar with BmNPV are shown in Table 1.

**Table 1**

| Feed required for infecting 1,000 5th-instar silkworms | | | |
|---|---|---|---|
| Feed sample | Amount of feed (g) | Time for consuming feed (hr) | Remarks |
| Tinopal-containing feed | 1400 | 24 | Containing 0.3% Tinopal |
| Feed for inoculating virus | 600 | 6 | Coated with 50 ml of 0.1% body fluid of silkworm with nuclear polyhedrosis virus |
| Synthetic feed | 8700 | 96 | Covered to prevent creeping out of silkworms |

Four days later, the body fluid was taken from each of silkworms and whether or not silkworms were infected with BmNPV was examined. In this case, silkworms fed with a normal feed in place of the feed containing Tinopal and feed coated with BmNPV were simultaneously reared as a control group. Table 2 shows the results after growth.

**Table 2**

| Test results for 1000 silkworms reared | | | |
|---|---|---|---|
| District | Silkworm weight at 24 hours later* | Silkworm weight at 96 hours later* | Rate of infection |
| Test district (1,000 silkworms) | 31.8 | 75.7 | 100 |
| Control district (50 silkworms) | 34.6 | 85.2 | - |

| | | | |
|---|---|---|---|
| *: denotes the weight of 20 silkworms Race of silkworms tested: Japanese No. 137 × China No. 146, 5th instar Feeding times after BmNPV inoculation: once | | | |

As shown in the Table, all silkworms in the test district were infected with BmNPV.

### Industrial Applicability

As shown in Examples, the silkworms fed with the synthetic feed containing Tinopal of the present invention before viral infection are susceptible to viral infection. Therefore, a large number of silkworms can be efficiently infected with a virus at a time by the method of the present invention.

All publications cited herein are incorporated in the present specification by references in its entirety. Furthermore, those skilled in the art may easily understand that various modifications and changes may be made without departing from the technical idea and scope of the present invention recited in the scope of the claims attached hereto. The present invention contemplates including such modifications and changes.

## Claims

1. A process for producing a feed for elevating a rate of infection with a recombinant virus in silkworms, comprising: mixing a powdery synthetic feed with distilled water in which Tinopal UNPA-GX is dissolved at a final concentration of 0.2 to 0.4% by weight of the feed; grinding by stirring; putting the mixture into a heat-resistant polyethylene bag; and heating the mixture in an autoclave.

2. The process for producing a feed for elevating a rate of infection with a recombinant virus in silkworms according to claim 1, comprising: mixing a powdery synthetic feed with distilled water in which Tinopal UNPA-GX is dissolved at a final concentration of 0.3% by weight of the feed; grinding by stirring; putting the mixture into a heat-resistant bag; and heating the mixture in an autoclave.

3. The process for producing a feed for elevating a rate of infection with a recombinant virus in silkworms according to claim 1 or 2, wherein the heating in an autoclave is performed at 100 to 121°C for 10 to 30 minutes.

4. A method of simultaneously inoculating orally a recombinant virus into a large number of silkworms reared on a silkworm basket, comprising: feeding a large number of 5th instar silkworms reared on a silkworm basket with the feed produced by the process according to claim 1; and mounting, at 20 to 24 hours later, a net carrying a feed coated with a recombinant virus-containing solution thereon, on the silkworm basket, thereby giving the feed coated with a recombinant-virus containing solution.

5. The method of simultaneously inoculating orally a recombinant virus into a large number of silkworms according to claim 4, comprising transferring silkworms fed with a recombinant virus and placed on the net onto a normal feed, together with the net.
